# EUROPÄISCHE PATENTANMELDUNG

(11) **EP 4 180 411 A1**
(43) Veröffentlichungstag der Anmeldung: **17.05.2023**
(21) Anmeldenummer: 21208227.5
(22) Anmeldetag: 15.11.2021
(51) Int. Cl.: C07C 45/50, C07C 47/02

(54) **VERFAHREN ZUR HERSTELLUNG VON ISO-ALDEHYDEN AUS N-OLEFINEN UNTER EINSATZ VON TRICYCLOHEXYLPHOSPHIN**

(71) Anmelder: Evonik Operations GmbH, 45128 Essen (DE)
(72) Erfinder: SALE, Anna Chiara, 45657 Recklinghausen (DE); FRANKE, Robert, 45772 Marl (DE); BRÄCHER, Alexander, 45721 Haltern am See (DE); FRIDAG, Dirk, 45721 Haltern am See (DE); KNOSSALLA, Johannes, 46514 Gahlen (DE); KUCMIERCZYK, Peter, 44628 Herne (DE); MARKOVIC, Ana, 45721 Haltern am See (DE)
(74) Vertreter: Evonik Patent Association

(57) **Zusammenfassung**

Verfahren zur Herstellung von *iso*-Aldehyden aus *n*-Olefinen unter Einsatz von Tricyclohexylphosphin.

## Beschreibung

Die Erfindung betrifft ein Verfahren zur Herstellung von *iso*-Aldehyden aus n-Olefinen unter Einsatz von Tricyclohexylphosphin.

2-Methylbutanal ist ein industriell gefragter Stoff. Er dient als Ausgangssubstanz für beispielsweise Alkoholen, Säuren, Estern und Aminen. So wird 2-Methylbutanal zum Beispiel für die Herstellung von Geruchs- und Aromastoffen verwendet.

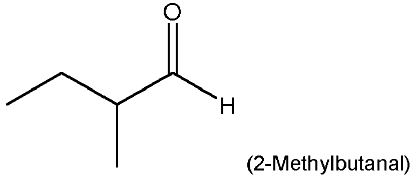

2-Methylbutanal kann durch die Hydroformylierung von 2-Buten hergestellt werden. Aufgrund der Symmetrie von 2-Buten spielt es keine Rolle, an welchem Ende der Doppelbindung die Aldehydgruppe ausgebildet wird, da immer 2-Methylbutanal entsteht.

Anders ist dies jedoch bei 1-Buten. Hier kann es zur Ausbildung es n-Aldehyds oder des *iso-*Aldehyds kommen. Oft stellt hierbei der n-Aldehyd das Hauptprodukt der Hydroformylierungsreaktion dar.

Die technische Aufgabe der Erfindung ist die Bereitstellung eines Verfahrens mit welchem, ausgehend von einem n-Olefinen, eine gute Ausbeute an *iso*-Aldehyd erzielt werden kann.

Die Aufgabe wird gelöst durch ein Verfahren gemäß Anspruch 1.

Verfahren umfassend die Verfahrensschritte:
a) Vorlegen einer Verbindung gemäß Formel (1): 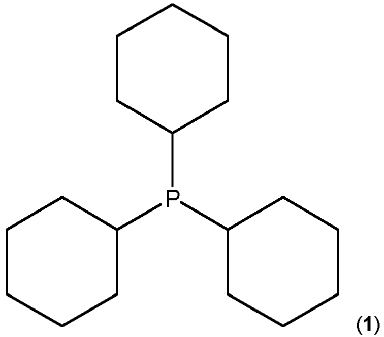 und von Rh(acac)(CO)₂ in einem Lösungsmittel, so dass ein Reaktionsgemisch entsteht;
b) Zugabe eines *n*-Olefins zu dem Reaktionsgemisch;
c) Zuführen von H₂ und CO, wobei das Zuführen bei einem Druck erfolgt im Bereich von 1 MPa (10 bar) bis 3 MPa (30 bar);
d) Erwärmen des Reaktionsgemisches aus a) bis c), wobei das *n*-Olefin zu einem *iso*-Aldehyd umgesetzt wird.

In einer Variante des Verfahrens erfolgt das Zuführen in Verfahrensschritt c) bei einem Druck im Bereich von 1,5 MPa (15 bar) bis 2,5 MPa (25 bar).

In einer Variante des Verfahrens erfolgt das Erwärmen in Verfahrensschritt d) auf eine Temperatur im Bereich von 100 °C bis 140 °C.

In einer Variante des Verfahrens erfolgt das Erwärmen in Verfahrensschritt d) auf eine Temperatur im Bereich von 110 °C bis 130 °C.

In einer Variante des Verfahrens wird die Rh-Verbindung in einer Menge bezogen auf das *n*-Olefin zugegeben im Bereich von 10 ppm bis 250 ppm.

In einer Variante des Verfahrens ist das *n*-Olefin ausgewählt aus: 1-Propen, 1-Buten, 1-Penten, 2-Methyl-1-buten, 3-Methyl-1-buten, 1-Hexen, 2-Methyl-1-hexen, 3-Methyl-1-hexen, 2-Ethyl-1-hexen, 1-Heptene, 2-Methylheptene, 3-Methylheptene, 1-Octene, 2-Methylocten, 3-Methylocten, 2-Ethyl-1-octen, 1-Nonen.

In einer Variante des Verfahrens ist das Lösungsmittel Toluol.

Im Folgenden soll die Erfindung anhand von Ausführungsbeispielen näher erläutert werden.

### Katalyseversuche

Die Hydroformylierung wurde in einem mit Druckkonstanthaltung, Gasflussmessung, und Begasungsrührer ausgestatteten 16 ml-Autoklaven der HEL group, Hertfordshire, Großbritannien, durchgeführt. Das Substrat (n-Octen) wurde mehrere Stunden über Natrium am Rückfluss erhitzt und unter Argon destilliert.

Für die Versuche wurden die Reaktionslösungen vorab unter Argonatmosphäre vorbereitet. Hierfür wurden 0,0021 g Rh(acac)(CO)₂ und die entsprechende Menge an Phosphitverbindung (L:Rh = 50:1) eingewogen und mit 8,0 ml Toluol aufgefüllt. Die jeweils eingebrachte Masse an Toluol wurde für die GC-Analyse bestimmt. Anschließend wurden 1,80 g n-Octen (16 mmol) hinzugegeben. Die vorbereiteten Lösungen wurden anschließend in den Autoklaven eingefüllt und dieser dreimal mit Argon und dreimal mit Synthesegas (Linde; H₂ (99,999 %) : CO (99,997%) = 1:1) gespült. Dann wurde der Autoklav bei einem Gesamtdruck von 10 bar unter Rühren (900 U/min) auf die angestrebte Temperatur aufgeheizt. Nach Erreichen der Reaktionstemperatur wurde der Synthesegasdruck auf 20 bar erhöht und die Reaktion bei konstantem Druck für 1 h geführt. Der Autoklav wurde nach Ablauf der Reaktionszeit auf Raumtemperatur abgekühlt, unter Rühren entspannt und mit Argon gespült. Jeweils 0,5 ml der Reaktionsmischungen wurden nach Beenden der Reaktion entnommen, mit 4 ml Pentan verdünnt und gaschromatographisch analysiert: HP 5890 Series II plus, PONA, 50 m × 0,2 mm × 0,5 µm. Die quantitative Bestimmung von Restolefin und Aldehyd erfolgte gegen das Lösungsmittel Toluol als interner Standard.

Der Versuch wurde mit den Verbindungen (1) und (2) durchgeführt.

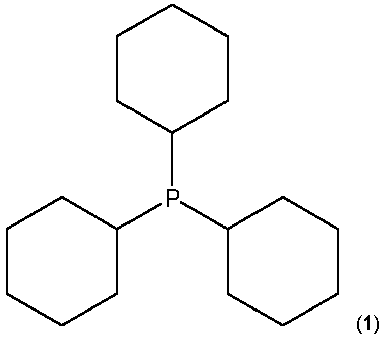

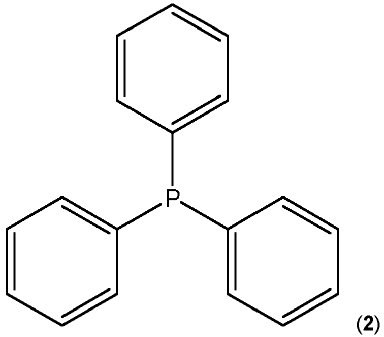

### Ergebnisse der Katalyseversuche

[Rh]: 100 ppm, p: 20 bar, T: 120 °C; t: 1 h

**Tabelle: Hydroformylierung von n-Octen**

| Ligand | Druck [bar] | Ausbeute [%] | *iso* [%] | *n* [%] |
|---|---|---|---|---|
| (**1**)* | 20 | 66 | 93,3 | 6,7 |
| (**2**) | 20 | 34 | 85,0 | 15,0 |

| | | | | |
|---|---|---|---|---|
| * erfindungsgemäßes Verfahren *iso*: Anteil des *iso*-Aldehyden an der erzielten Ausbeute n: Anteil des n-Aldehyden an der erzielten Ausbeute | | | | |

Die durchgeführten Versuche belegen, dass die gestellte Aufgabe durch ein erfindungsgemäßes Verfahren gelöst wird.

## Patentansprüche

1. Verfahren umfassend die Verfahrensschritte:
a) Vorlegen einer Verbindung gemäß Formel (1): 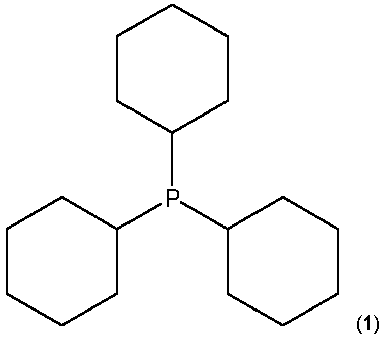 und von Rh(acac)(CO)₂ in einem Lösungsmittel, so dass ein Reaktionsgemisch entsteht;
b) Zugabe eines *n*-Olefins zu dem Reaktionsgemisch;
c) Zuführen von H₂ und CO, wobei das Zuführen bei einem Druck erfolgt im Bereich von 1 MPa (10 bar) bis 3 MPa (30 bar);
d) Erwärmen des Reaktionsgemisches aus a) bis c), wobei das *n*-Olefin zu einem *iso*-Aldehyd umgesetzt wird.

2. Verfahren nach Anspruch 1,
wobei das Zuführen in Verfahrensschritt c) bei einem Druck erfolgt im Bereich von 1,5 MPa (15 bar) bis 2,5 MPa (25 bar).

3. Verfahren nach einem der Ansprüche 1 bis 2,
wobei das Erwärmen in Verfahrensschritt d) auf eine Temperatur erfolgt im Bereich von 100 °C bis 140 °C.

4. Verfahren nach einem der Ansprüche 1 bis 3,
wobei die Rh-Verbindung in einer Menge bezogen auf das *n*-Olefin zugegeben wird im Bereich von 10 ppm bis 250 ppm.

5. Verfahren nach einem der Ansprüche 1 bis 4,
wobei das *n*-Olefin ausgewählt ist aus: 1-Propen, 1-Buten, 1-Penten, 2-Methyl-1-buten, 3-Methyl-1-buten, 1-Hexen, 2-Methyl-1-hexen, 3-Methyl-1-hexen, 2-Ethyl-1-hexen, 1-Heptene, 2-Methylheptene, 3-Methylheptene, 1-Octene, 2-Methylocten, 3-Methylocten, 2-Ethyl-1-octen, 1-Nonen.

6. Verfahren nach einem der Ansprüche 1 bis 5,
wobei das Lösungsmittel Toluol ist.

## Geänderte Patentansprüche

### Geänderte Patentansprüche gemäss Regel 137(2) EPÜ.

1. Verfahren umfassend die Verfahrensschritte:
a) Vorlegen einer Verbindung gemäß Formel (1): 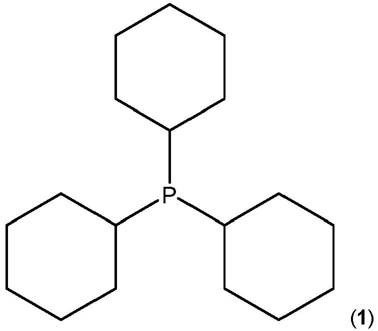 und von Rh(acac)(CO)₂ in einem Lösungsmittel, so dass ein Reaktionsgemisch entsteht;
b) Zugabe eines n-Olefins zu dem Reaktionsgemisch, wobei das n-Olefin ausgewählt ist aus: 1-Propen, 1-Buten, 1-Penten, 2-Methyl-1-buten, 3-Methyl-1-buten, 1-Hexen, 2-Methyl-1-hexen, 3-Methyl-1-hexen, 2-Ethyl-1-hexen, 1-Heptene, 2-Methylheptene, 3-Methylheptene, 1-Octene, 2-Methylocten, 3-Methylocten, 2-Ethyl-1-octen, 1-Nonen;
c) Zuführen von H₂ und CO, wobei das Zuführen bei einem Druck erfolgt im Bereich von 1 MPa (10 bar) bis 3 MPa (30 bar);
d) Erwärmen des Reaktionsgemisches aus a) bis c), wobei das n-Olefin zu einem iso-Aldehyd umgesetzt wird.

2. Verfahren nach Anspruch 1,
wobei das Zuführen in Verfahrensschritt c) bei einem Druck erfolgt im Bereich von 1,5 MPa (15 bar) bis 2,5 MPa (25 bar).

3. Verfahren nach einem der Ansprüche 1 bis 2,
wobei das Erwärmen in Verfahrensschritt d) auf eine Temperatur erfolgt im Bereich von 100 °C bis 140 °C.

4. Verfahren nach einem der Ansprüche 1 bis 3,
wobei die Rh-Verbindung in einer Menge bezogen auf das *n*-Olefin zugegeben wird im Bereich von 10 ppm bis 250 ppm.

5. Verfahren nach einem der Ansprüche 1 bis 4,
wobei das Lösungsmittel Toluol ist.
